# EUROPEAN PATENT SPECIFICATION

(11) **EP 1 342 085 B1**
(45) Date of publication and mention of the grant of the patent: **30.09.2009**
(21) Application number: 01997059.9
(22) Date of filing: 13.12.2001
(51) Int. Cl.: G01N 33/50

(54) **NON-APOPTOTIC FORMS OF CELL DEATH AND METHODS OF MODULATION**
NON-APOPTOTISCHE FORMEN DES ZELLTODS UND VERFAHREN ZUR MODULATION
FORMES NON APOPTOTIQUES DE MORT CELLULAIRE ET METHODES DE MODULATION

(30) Priority: 14.12.2000 US 256023 P; 19.12.2000 US 256694 P
(43) Date of publication of application: 10.09.2003
(73) Proprietor: The Burnham Institute, La Jolla, CA 92037 (US)
(72) Inventor: SPERANDIO, Sabina, San Diego, California 92130 (US); DE BELLE, Ian, La Jolla, CA 92037 (US); BREDESEN, Dale, E., Novato, CA 94949 (US)
(74) Representative: UEXKÜLL & STOLBERG
(86) International application number: PCT/US2001/048261
(87) International publication number: WO 2002/054066

(56) References cited:
- WO-A-00/21550
- JP-A- 63 218 681
- US-A- 5 958 872
- TURMAINE M ET AL: "NONAPOPTOTIC NEURODEGENERATION IN A TRANGENIC MOUSE MODEL OF HUNTINGTON'S DISEASE" PROCEEDINGS OF THE NATIONAL ACADEMY OF SCIENCES OF USA, NATIONAL ACADEMY OF SCIENCE. WASHINGTON, US, vol. 97, no. 14, 5 July 2000 (2000-07-05), pages 8093-8097, XP000990139 ISSN: 0027-8424
- RESNICOFF M ET AL: "The insulin -like growth factor I receptor protects tumor cells from apoptosis in vivo" CANCER RESEARCH, AMERICAN ASSOCIATION FOR CANCER RESEARCH, BALTIMORE, MD, US, vol. 55, no. 11, 1995, pages 2463-2469, XP002124515 ISSN: 0008-5472
- MAJNO G ET AL: "APOPTOSIS ONCOSIS AND NECROSIS AN OVERVIEW OF CELL DEATH" AMERICAN JOURNAL OF PATHOLOGY, PHILADELPHIA, PA, US, vol. 146, no. 1, January 1995 (1995-01), pages 3-15, XP000884170 ISSN: 0002-9440
- SPERANDIO SABINA ET AL: "An alternative, nonapoptotic form of programmed cell death." PROCEEDINGS OF THE NATIONAL ACADEMY OF SCIENCES OF THE UNITED STATES, vol. 97, no. 26, 19 December 2000 (2000-12-19), pages 14376-14381, XP002204088 December 19, 2000 ISSN: 0027-8424
- SPERANDIO S ET AL: "A proteomic approach to the characterization of non-apoptotic programmed cell death." SOCIETY FOR NEUROSCIENCE ABSTRACTS, vol. 27, no. 1, 2001, page 869 XP001083701 31st Annual Meeting of the Society for Neuroscience;San Diego, California, USA; November 10-15, 2001 ISSN: 0190-5295
- OPPENHEIM RONALD W ET AL: "Programmed cell death of developing mammalian neurons after genetic deletion of caspases." JOURNAL OF NEUROSCIENCE, vol. 21, no. 13, 1 July 2001 (2001-07-01), pages 4752-4760, XP001084981 ISSN: 0270-6474

## Description

### BACKGROUND OF THE INVENTION

The present invention relates generally to molecular medicine and drug screening assays and more specifically to interactions involved in modulating nonapoptotic programmed cell death and methods of identifying drugs that modulate nonapoptotic programmed cell death.

Apoptosis, or programmed cell death, is an active process that plays a major role during cellular differentiation, development of multicellular organisms, tissue homeostasis, metamorphosis and cancer. The original definition of apoptosis as a form of cell death distinct from necrosis was based on morphological criteria. Genetic studies in C. elegans led to the identification of key components of the apoptotic cell death pathway, including the protease Ced-3, which was found to have remarkable sequence similarity to interleukin-1β-converting enzyme (caspase-1), a mammmalian proteinase. Subsequent studies identified over a dozen caspase family members important for apoptosis.

Recently, it has become clear that the morphological descriptions of apoptosis and necrosis are not adequate to describe all cell deaths. In this regard, reports of various types of cell deaths that do not conform to the criteria for either of these forms of cell death have recently been published. These types of cell deaths that appear to be neither apoptotic nor necrotic include certain developmental cell deaths, such as "autophagic" cell death (see, Schweichel, Z Anat Entwicklungsgesch 136:192-203 (1972); Schweichel and Merker, Teratology 7:253-66 (1973); Schwartz, Bioessays 13:389-95 (1991); Clarke, Anat Embryol 181:195-213 (1990); and Lockshin and Williams, Insect Physiol, 10:643-9 (1964)) and "cytoplasmic" cell death (Schweichel and Merker, supra, 1973; Clarke, Anat Embryol 181:195-213 (1991); Pilar and Landmesser, J Cell Biol 68:339-56 (1976); Oppenheim, Annu Rev Neurosci 14:453-501 (1991); Oppenheim, Trends Neurosci 17:487-493 (1985); Cunningham, Int Rev Cytol 74:163-86 (1982)). Additional forms of cell death that neither fit the criteria of apoptosis nor those of necrosis include some neurodegenerative cell deaths, such as those in a transgenic mouse model of amyotrophic lateral sclerosis (Canto and Gurney, American Journal of Patholology 145:1271-1279 (1994)), as well as some ischemia-related cell deaths featuring cell swelling, referred to as "oncosis" (Majno and Joris,, Am J Pathol 146:3-15 (1995)).

The biochemical basis for these alternative morphological forms of cell death remains unknown. There exists a need for understanding the mechanisms for nonapoptotic forms of programmed cell death, which is implicated in developmental cell death, neural cell death diseases and cancer, and for the design of novel therapeutic agents that can modulate these alternative forms of cell death. For example, JP-A-63218681 discloses that oligomycin can be used as an anticancer agent. The present invention satisfies this need and provides related advantages as well.

### SUMMARY OF THE INVENTION

The invention is directed to methods for identifying molecules that modulate paraptosis, a newly identified nonapoptotic form of programmed cell death. The invention also provides a method of inhibiting paraptosis by preventing association of caspase-9 and an endogenous paraptosis-mediating molecule. In one embodiment, the invention provides a method of inhibiting paraptosis by preventing association of caspase-9 and the Insulin-Like Growth Factor I Receptor (IGFIR). Also provided by the present invention are methods of identifying endogenous paraptosis-mediating molecules.

### BRIEF DESCRIPTION OF THE DRAWINGS

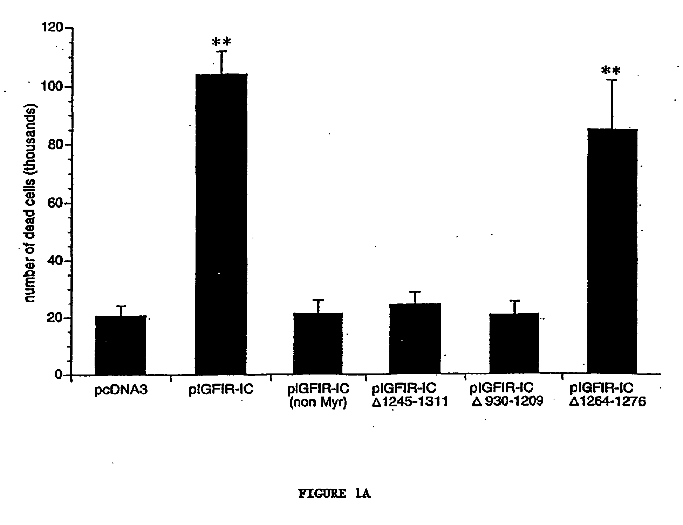

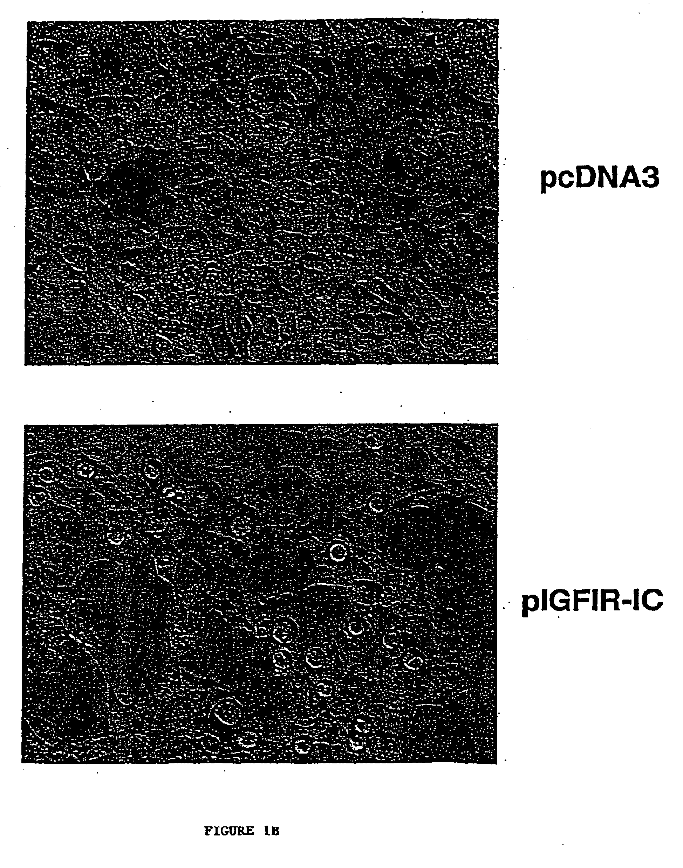

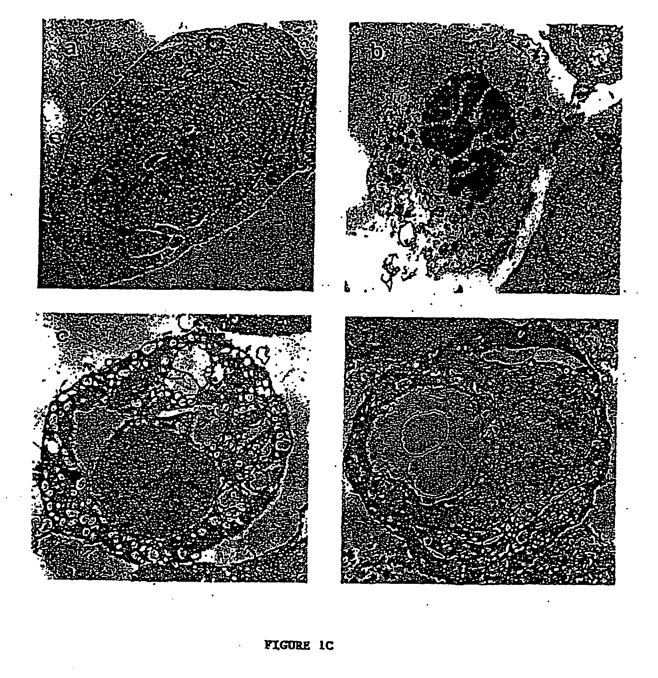

Figure 1 shows (A) a comparison between the cell death induced by pIGFIR-IC and non- myristylated form of the IC domain and deletion mutants (Asterisks indicate a highly significant difference from the control as determined by one-way ANOVA (F=63.3; P<0.0001); P<0.0001 for pIGFIR-IC and for pIGFIR-ICD1264-1276, Bonferroni/Dunn post-hoc test. Error bars represent the SEM from three independent experiments). Panel (B) shows light microscopy images, using Hoffman optics, demonstrating morphological changes induced by IGFIR-IC. Panel (C) shows ultrastructural characteristics of IGFIR-induced cell death. 293T cells were transfected with control empty vector pcDNA3 (a), pC9 (caspase-9) (b), or pIGFIR-IC (c,d). While caspase-9 transfected cells displayed characteristic features of apoptosis, including chromatin condensation, IGFIR-IC transfected cells did not. In contrast, extensive cytoplasmic vacuolization was observed in the absence of nuclear fragmentation, cellular blebbing, or apoptotic body formation. Note that the cell shown in b displays both the chromatin condensation characteristic of apoptosis and membrane disruption characteristic of necrosis, suggesting secondary necrosis following apoptosis. Original magnification: 6000X.

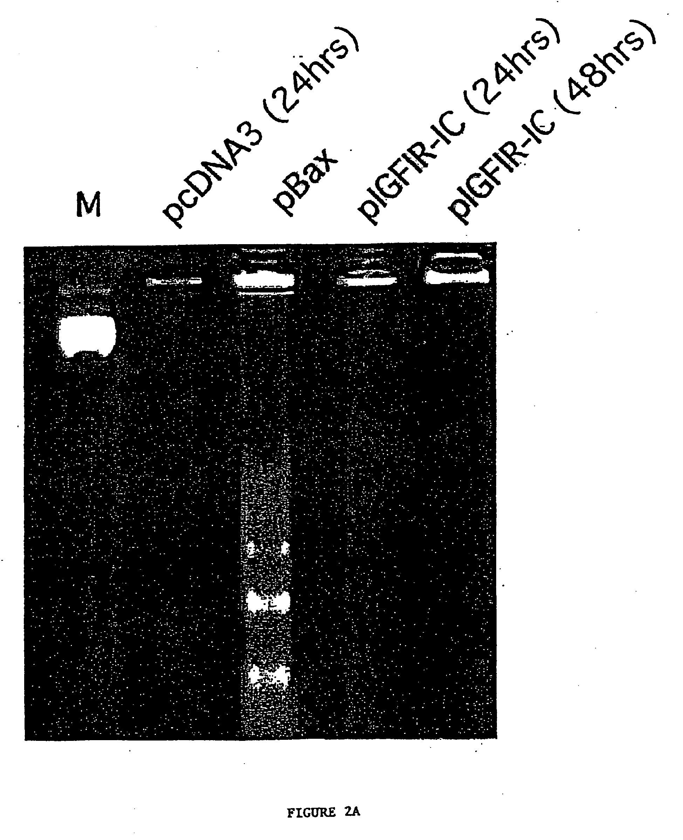

Figure 2(A) shows internucleosomal DNA fragmentation of cells transfected as described in the legend to Figure 1. Soluble DNA was extracted from cells 24 or 48 hours after transfection. Whereas the Bax-transfected cells demonstrated internucleosomal DNA fragments, the chromatin in IGFIR-IC transfected cells remained intact at 24 and 48 hours following transfection. Panel (B) shows TUNEL staining of cells transfected as in A. Cells were assayed 48 hours following transfection. The upper panels show FITC staining of the TUNEL labeling. Bax, but not IGFIR-IC, transfected cells show TUNEL positive staining. The lower panels show the same fields of cells stained with propidium iodide. Cells were permeabilized prior to staining. Similar results were obtained at 24 hours. Panel (C) shows that transcription and protein synthesis are required for IGFIR-induced cell death. Actinomycin D or cycloheximide (numbers represent µg/ml) were added 8.5 hrs after the transfections and the medium was replaced after 12 hrs with fresh medium without drugs. Floating cells were collected after 24 hrs and counted in the presence of trypan blue. Control samples were treated with solvent only (ethanol 0.05 %). The expression of IGFIR-IC at the time of administration of the drugs was comparable to the expression at 24 h, as assessed by Western blot analysis. The error bars represent the SEM of 4 independent experiments. Within the samples transfected with pIGFIR-IC, the asterisks indicate a highly significant difference (P = 0.0011 for 0.25 mg/ml CHX, and P<0.0001 for 0.5 mg/ml CHX, and 0.5 mg/ml and 1 mg/m Act.D; Bonferroni/Dunn post-hoc test) from the pIGFIR-IC transfected sample treated with solvent only (EtOH), as determined by one-way ANOVA (F=20.727; P<0.0001).

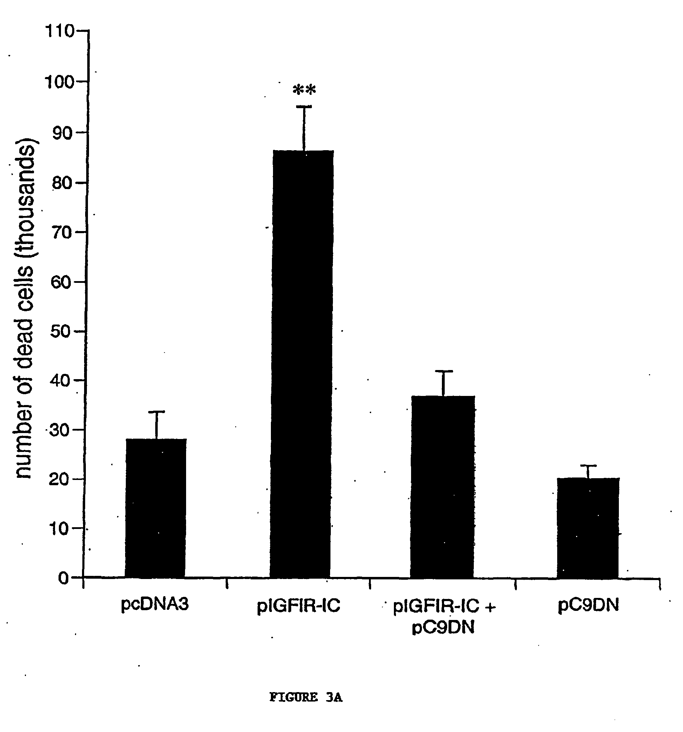

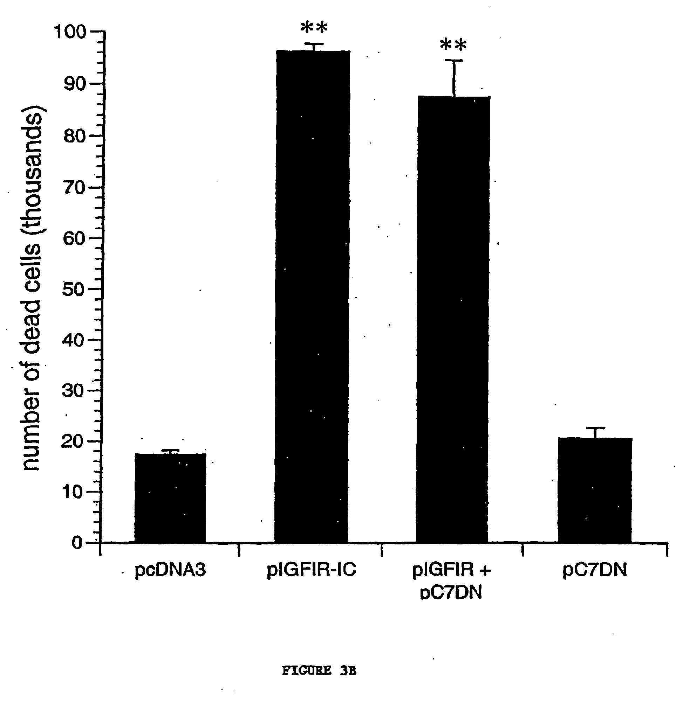

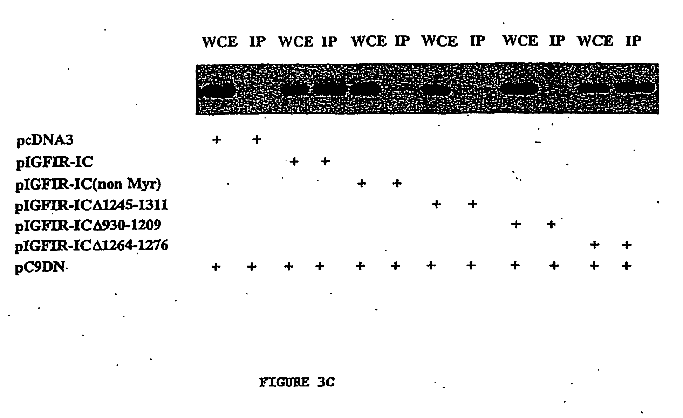

Figure 3 shows IGFIR=IC-induced cell death is blocked by a catalytic mutant of pro-caspase-9. As shown in panels (A) and (B) 293T cells were transfected with 2µg of DNA consisting of either pcDNA3, 0.5 µg pIGFIR-IC + 1.5 µg pcDNA3, or 0.5 µg pIGFIR-IC + 1.5 µg dominant negative caspase-9 (pC9DN, in A) or dominant negative caspase-7 (pC7DN, in panel B) expression constructs. 48 hours after transfection, dead cells were counted. Asterisks denote a highly significant difference from the control as determined by one-way ANOVA. In panel (A) F=25.2, P=0.0002 with P=0.0001 for pIGFIR-IC (Bonferroni/Dunn post-hoc test). In panel (B) F=118.487, P<0.0001, with P<0.0001 for pIGPIR-IC and pIGFIR-IC+pC9DN (Bonferroni/Dunn post-hoc test). Error bars represent the SEM from three independent experiments. Panel (C) showa protein extracts from cells cotransfected with pIGFIR-IC wild-type or mutants, as indicated, and FLAG-tagged C9DN expression constructs were subjected to immunoprecipitation using an anti-IGFIR polyclonal antibody. Immunoprecipitates were then resolved by 15% SDS-PAGE and Western blotting was performed using a monoclonal antibody against the FLAG epitope to detect caspase-9. WCE=whole cell extract, IP=immunoprecipitation.

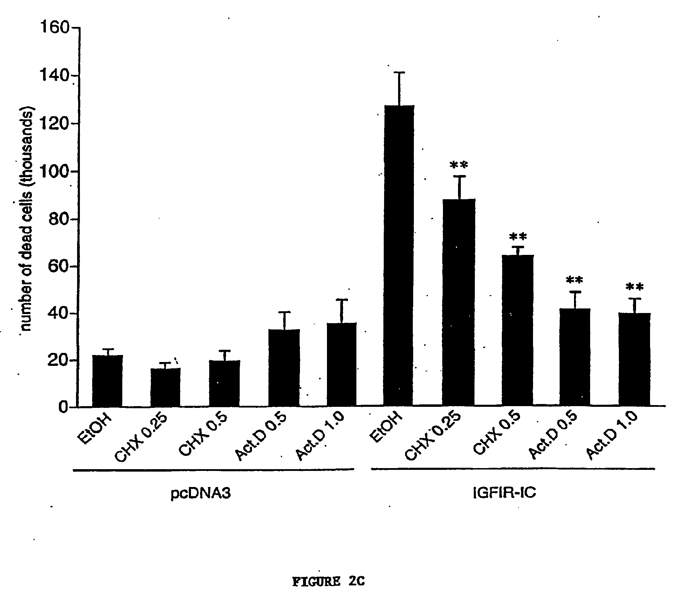

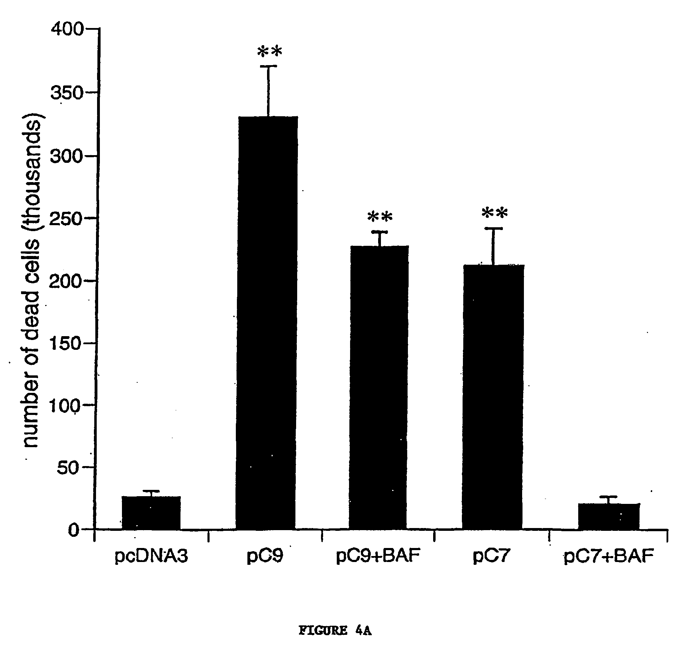

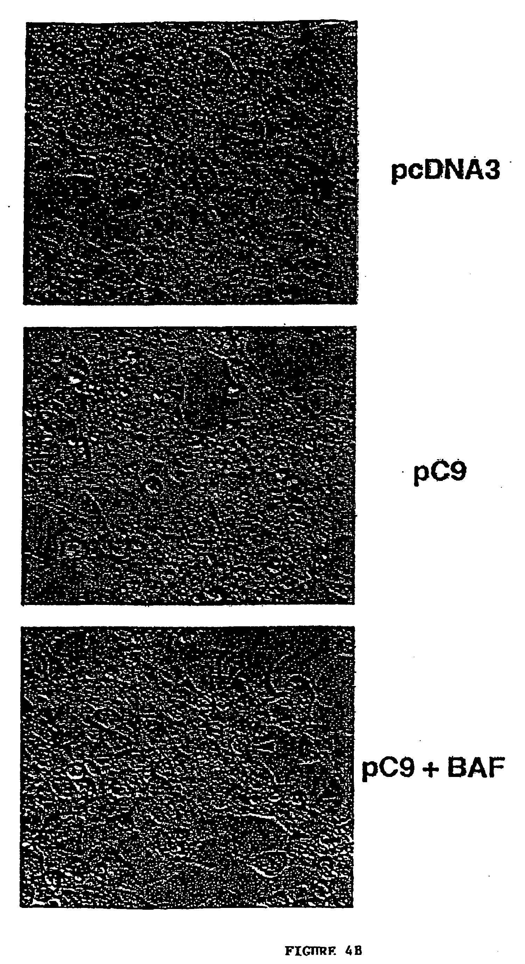

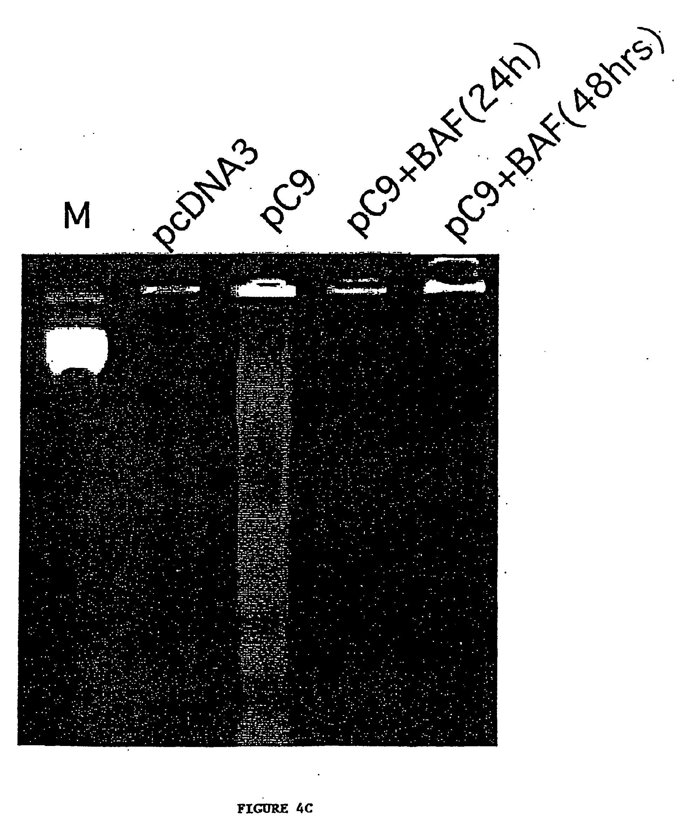

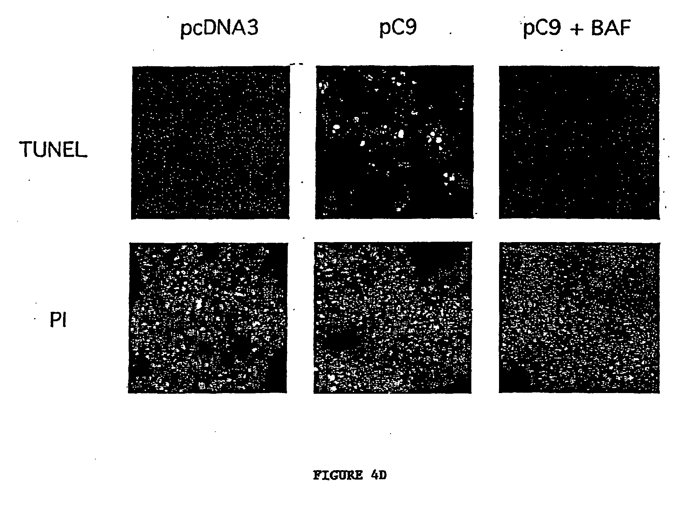

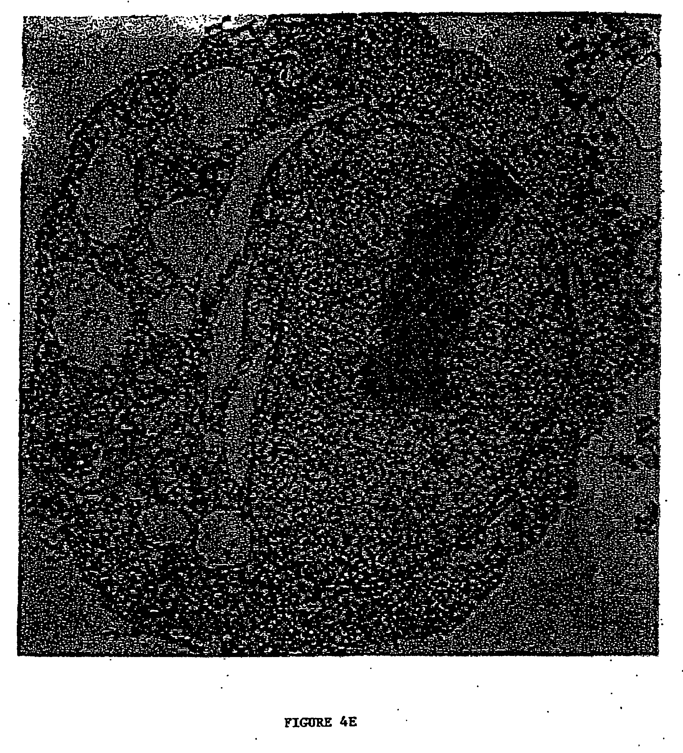

Figure 4 shows that caspase-9 induces both apoptosis and non-apoptotic cell death. In panel (A) 293T cells were transfected with the indicated pcDNA3-based caspase zymogen expression constructs. The general caspase inhibitor BAF was added at 50 µM final concentration. Cell death was assayed 48 hours after transfection. While BAF was able to inhibit caspase-7 induced cell death completely, it only inhibited caspase-9 induced cell death partially (30%). Asterisks indicate a highly significant difference from the control as determined by one-way ANOVA analysis (F=34.624, P<0.0001), P<0.0001 for pC9, P=0.0002 for pC7, and P=0.0001 for pC9+BAF (Bonferroni/Dunn post-hoc test). Error bars represent the SEM from three independent experiments. Panel (B) shows light microscopy images of 293T cells transfected with control vector (pcDNA3), pro-caspase-9 alone (pC9), or in the presence of 50 µM BAF (pC9 + BAF). Cells were photographed 24 hours after transfection. Expression of pro-caspase-9 resulted in the induction of a typical apoptotic phenotype, including cell shrinkage and the formation of apoptotic bodies (center panel). BAF suppressed the apoptotic phenotype, but cells displayed a vacuolated morphology similar to that observed with IGFIR-IC-induced cell death (lower panel, arrowheads). Panel (C) shows internucleosomal DNA fragmentation of 293T cells transfected with the indicated pro-caspase expression constructs alone, or in the presence of 50 µM BAF. Internucleosomal DNA fragmentation was completely inhibited by BAF. Panel (D) shows TUNEL staining of 293T cells 48 hours after transfection with the indicated constructs. Cells were stained as described in Fig. 2C. Panel (E) Electron micrograph of 293T cells transfected with an expression construct for pro-caspase-9 in the presence of zVAD.fmk (40 µM). Morphology similar to IGFIR-IC-induced cell death was observed, including cytoplasmic vacuolation and the absence of chromatin condensation, nuclear fragmentation, and apoptotic body formation. Original magnification: 6000X.

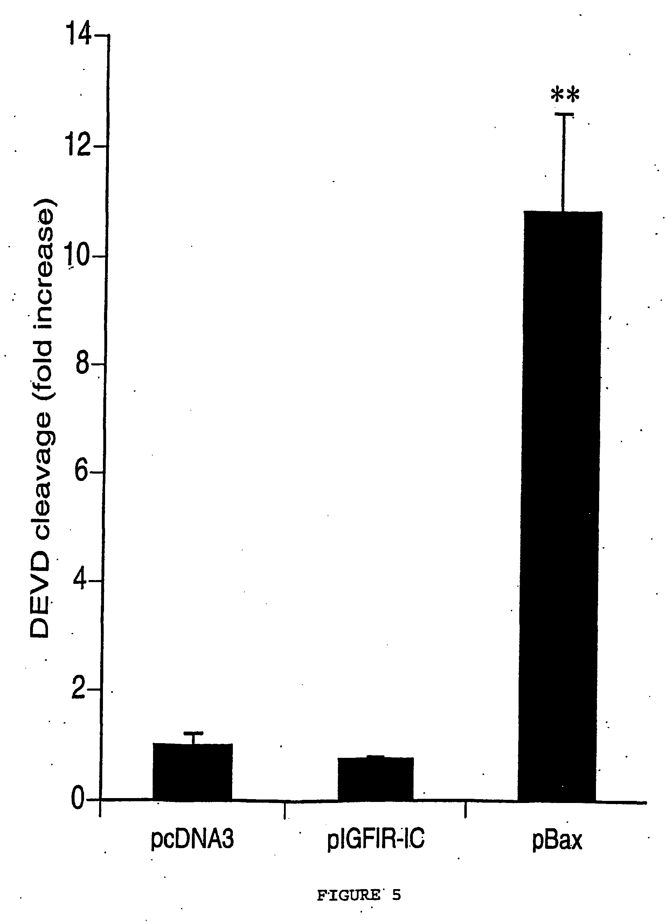

Figure 5 shows that IGFIR-IC induces cell death in absence of caspase-3 activation. In panel (A) caspase-3 activity was measured 48 hours following transfection of 293T cells with the indicated expression constructs. Expression of Bax, but not IGFIR-IC, resulted in the induction of caspase-3 activity. Asterisks indicate a highly significant difference from the control determined by one-way ANOVA (F=30. 862; P<0.0.007); P< 0.0005 for Bax compared to control and IGFIR-IC, Bonferroni/Dunn post-hoc test. Error bars represent the SEM from three independent experiments. Similar results were obtained when the caspase assays were performed 24 hours after transfection.

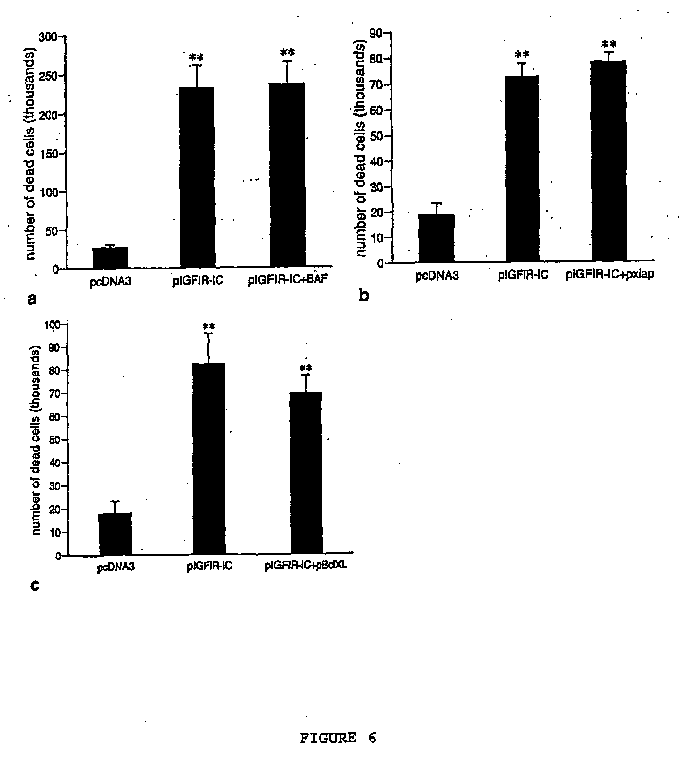

Figure 6 shows IGFIR-induced cell death is not blocked by caspase inhibitors. Cell death counts were performed following IGFIR-IC transfection in 293T cells. Neither the administration of the caspase inhibitor BAF at 50 mM (A) nor the cotransfection of xiap (B) or Bcl-xL (C) were effective in inhibiting the cell death. In panels (B) and (C) 0.5 mg of pIGFIR-IC were cotransfected with 1.5 mg of either pcDNA3 or pxiap or pBcl-xL. The asterisks indicate a highly significant difference from the controls (pcDNA3) as determined by one-way ANOVA analysis. In panel (A), F=25.377, P=0.0012 with P=0.0009 for IGFIR-IC and P=0.0008 for pIGFIR-IC+BAF (Bonferroni/Dunn post-hoc test). In panel (B), F=53.372, P=0.0002, with P=0.0001 for pIGFIR-IC and P<0.0001 for pIGFIR-IC+pxiap (Bonferroni/Dunn post-hoc test). In panel(C), F=13, P=0.022 with P=0.0009 for pIGFIR-IC and P=0.004 for pIGFIR-IC+pBcl-xL (Bonferroni/Dunn post-hoc test). Error bars represent the SEM from 3 independent experiments.

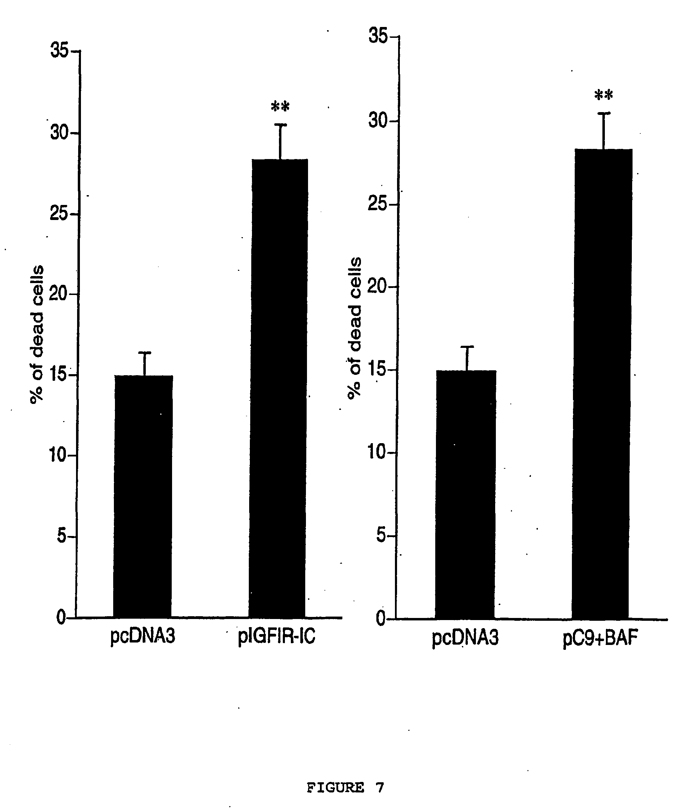

Figure 7 shows Apaf-1 is not required for IGFIR induced cell dearth. Cell death induction in mouse embryo fibroblasts derived from Apaf-1 null mice, after transfection with vector alone (pcDNA3), IGFIR-IC (left panel) or control (pcDNA3) and Caspase 9 in the presence of BAF (right panel). Total cells were collected and cell death was assessed by trypan blue exclusion and expressed as the number of dead cells over the total number of cells. Error bars represent the SEM of three independent experiments. Asterisks indicate a highly significant difference from the controls determined by Student's t test (two tailed; p< 0.007 for pIGFIR-IC; p< 0.0007 for pC9+BAF).

### DETAILED DESCRIPTION OF THE INVENTION

In accordance with the present invention, there is provided a method for identifying a paraptosis-modulating compound by (a) inducing paraptosis in a cellular system; (b) contacting the cellular system with a test-compound; and (c) identifying a compound that modulates paraptosis, wherein said paraptosis in induced by upregulating caspase-9 in the presence of an apoptosis inhibitor, In a related embodiment, the invention provides a method of identifying a paraptosis-modulating compound by (a) inducing paraptosis in a first cellular system and inducing paraptosis in a second cellular system; (b) contacting the first cellular system with a test-compound and contacting said second cellular system with a control compound; and c) comparing the amount of cell death between the first and said second cellular system, where a difference in the amount of cell death between the first and second cellular system indicates that the test compound modulates paraptosis, wherein said paraptosis in said first and said second cellular system is induced by upregulating caspase-9 in the presence of an apoptosis inhibitor.

As used herein the term "paraptosis" refers to a newly identified form of programmed cell death mediated by caspase-9 that lacks many of the molecular, biochemical and cytological characteristics of apoptosis. As described herein, the mediation of paraptosis is a newly identified function of caspase-9 that is distinct from the role of caspase-9 in the apoptotic pathway as evidenced, for example, by the Apaf-1 independence of the paraptotic pathway. For example, nuclear fragmentation, apoptotic body formation and chromatin condensation are ultrastrucural features of apoptosis that are not observed or are greatly reduced in paraptosis. In addition, paraptosis can be associated with cytoplasmic vacuolation. Inhibition of apoptosis by caspase inhibitors zVAD.fmk, BAF, p35m, X-chromosome-linked inhibitor of apaoptosis (xiap), and Bcl-2 family member Bcl-x_{L}. are features associated with apoptosis that are not observed or greatly reduced in non-apoptotic cell death. A further feature of apoptosis not associated with paraptosis is the dependence on Apaf-1, the cytosolic cofactor of caspase-9 zymogen. In addition to being distinct from apoptotic cell death, paraptosis is further distinct from necrosis. For example, cleavage of poly(ADP-ribose)polymerase (PARP) yielding distinctive 50 to 62 kDa fragments is a feature of necrosis that is not observed in nonapoptic cell death. Thus, paraptosis is a nonapoptotic form of programmed cell death.

As used herein, the term "paraptosis-modulating compound" refers to a compound that has the ability to increase, decrease or otherwise modify the rate or amount of nonapoptotic cell death. A paraptosis-modulating compound can act by altering directly or indirectly the severity, extent, intensity, magnitude, duration or frequency of cell death. A paraptosis-modulating compound can also, for example, alter the severity, extent, intensity, magnitude, duration or frequency of a symptom associated with paraptosis. A paraptosis-modulating compound can modulate paraptosis by altering the expression of an endogenous paraptosis-mediating molecule such as, for example, caspase-9 or IGFIR. An "endogenous paraptosis-mediating molecule," as used herein, is a molecule that plays a role in the molecular pathway that regulates paraptosis *in vivo*. The role of an endogenous paraptosis-mediating molecule can be that of a positive or a negative regulator. Examples of endogenous paraptosis-mediating molecules include, for example, caspase-9, IGFIR, and the like. The caspase-9 nucleic acid and amino acid sequences are well known in the art, and are set forth at accession XM_001584 (human) on Gen Bank. The IGFIR nucleic acid and amino acid sequences are described, for example, by Ullrich et al., EMBO J 5(10):2503-2512(1986).

As used herein, the term "cellular system" refers to an in vitro cellular system such as a cell culture system. Furthermore, the terms "first cellular system" and "second cellular system" refer to identical cellular systems with regard to all components and conditions, except that the first cellular system is contacted with the test compound and the second cellular system is contacted with a biologically inactive control-compound. In other words, the only difference between the first and second cellular systems is that the first cellular system is contacted with the test compound.

The invention provides a method for identifying a paraptosis-modulating compound. The paraptosis-modulating compounds identified by the methods of the invention have therapeutic value for a variety of disorders associated with paraptosis. For example, the paraptosis modulators identified by the invention methods can be used to treat those types of neurodegeneration associated with nonapoptotic cell death such as, for example, familial amyotrophic lateral sclerosis as described by Canto and Gurney, American Journal of Patholology 145:1271-1279 (1994); Huntington's Disease as described by Turmaine et al., Procl. Nat. Acad. Sci. USA, 97:8093-8097 (2000). Notably, there is no evidence that the majority of neural cell death in other neurodegenerative diseases including Parkinson's Disease and Alzheimer's Disease is apoptotic in nature.

As described herein, Insulin-Like Growth Factor I Receptor (IGFIR), as well as the IGFIR intracytoplasmic domain (IGFIR-IC), are paraptosis-mediating molecules that induce a form of non-apoptotic programmed cell death characterized by cytoplasmic vacuolation and resistance to apoptosis inhibitors. This form of cell death, designated paraptosis, requires transcription and de novo protein synthesis. In addition, a microarray screening comparing gene expression profiles between IGFIR induced cell death and apoptotic cell death showed that fewer than 2% of those genes that are differentially expressed are shared between the two cell death programs, which is consistent with distinct cell death programs. Overexpression of fragments of the intracellular domain of IGFIR in cancer cells has been shown to reduce tumorigenicity in nude mice and induce cell death (see, Hongo et al., Cancer Research 58:2477-2484 (1998); Liu et al., Cancer Research 58:570-576 (1998)). The expression of IGFIR is decreased in prostate cancer (Tennant et al., J Clin. Endocrinol. Metab. 81:3774-82 (1996)), and its reexpression in immortalized human prostate cells inhibited the malignant phenotype (Plymate et al., Endocrine 7:119-24 (1997)). Moreover, a potential role for IGFIR in developmental cell death is suggested by the phenotype of IGFIR-null mice, which includes a higher neuronal density in the brain stem and spinal cord (Liu et al. Cell 75:59-72 (1993)). As described herein, the paraptotic form of programmed cell death induced by IGFIR and IGFIR-IC is distinct from apoptosis based on morphological, biochemical and molecular features.

Paraptosis is a nonapoptotic form of programmed cell death that can be induced by IGFIR and is mediated by a newly identified function of caspase-9 that is distinct from the role of caspase-9 in the apoptotic pathway. Thus, caspase-9 participates in both, apoptotic and paraptotic forms of cell death. However, caspase inhibitors that have been shown to inhibit apoptosis fail to inhibit paraptosis, an indication that distinct catalytic activities of caspase-9 mediate apoptosis and paraptosis. IGFIR coimminoprecipitates with caspase-9 and mutants of IGFIR that fail to coimmunoprecipitate with caspase-9 also fail to induce paraptosis. Thus, one method of inhibiting paraptosis contemplated by the present invention is by preventing the association of caspase-9 and IGFIR. Furthermore, the invention contemplates methods of inhibiting paraptosis by preventing association of caspase-9 and an endogenous paraptosis-mediating molecule. In this regard, the invention provides methods of identifying an endogenous paraptosis-mediating molecule by inducing paraptosis in a cellular system and identifying a molecule that associates with caspase-9, wherein said paraptosis is induced by upregulating caspase-9 in the presence of an apoptosis inhibitor.

The term "upregulating, as used herein in reference to a caspase refers to effecting an increase in the amount, expression or activity level of caspase-9 or caspase-9 zymogen that, in the absence of apoptosis inhibitors, is associated with induction of apoptosis. The upregulation of caspase-9 can be direct as, for example, by increasing the abundance of caspase-9 or caspase-9 zymogen or by increasing the amount, expression or activity level of any molecule that activates caspase-9 or caspase-9 zymogen.

The pro-apoptotic and the pro-paraptotic effects of caspase-9 can be distinguished by the lack of effect of caspase inhibitors including, for example, BAF, zVAD.fmk, p35, and xiap on paraptosis; lack of a requirement of paraptosis for activation of procaspase-9 zymogen by Apaf-1; and lack of suppression of paraptosis by mutation catalytic sites of caspase-9. Thus, as described herein, caspase-9 displays at least two distinct activities, one that is pro-apoptotic and one that induces nonapoptotic cell death, designated paraptosis.

The term "preventing," as used herein in reference to the complex formation between IGFIR and caspase-9, refers to a decrease in the affinity of association. Agents that can alter the association of IGFIR and caspase-9 can be useful for modulating paraptosis in a population of cells and, therefore, can be useful as medicaments for treating a pathology characterized by an aberrant level of paraptosis. Such an agent can be, for example, an anti-idiotypic antibody, which can inhibit the association of a IGFIR and caspase-9.

The method of the invention for identifying a molecule that modulates paraptosis involves inducing paraptosis in a cellular system by upregulating caspase-9 in the presence of an apoptosis inhibitor. As described herein, the expression of caspase-9 in the presence of the apoptosis inhibitor BAF (or zVAD.fmk) induces paraptosis. Thus, paraptosis can be induced by upregulating caspase-9 in the presence of an apoptosis inhibitor. Similarly, upregulation of caspase-2, caspase-7 or caspase-8, will include both paraptosis and apoptosis. In addition, paraptosis can be induced by environmental stimuli such as, for example, heatshock, and the like.

Once paraptosis has been induced in a cellular system, the invention method contemplates contacting the cellular system with a test-compound to identify an agent that modulates paraptosis. The methods provided by the invention are particularly useful to identify, from among a diverse population of molecule, those agents that modulate paraptosis. Methods for producing libraries containing diverse populations of molecules, including chemical or biological molecules such as simple or complex organic molecules, peptides, proteins, peptidomimetics, glycoproteins, lipoproteins, polynucleotides, and the like, are well known in the art (Huse, U.S. Patent No. 5,264,563, issued November 23, 1993; Blondelle et al., Trends Anal. Chem. 14:83-92 (1995); York et al., Science 274:1520-1522 (1996); Gold et al., Proc. Natl. Acad. Sci., USA 94:59-64 (1997); Gold, U.S. Patent No. 5,270,163, issued December 14, 1993). Such libraries also can be obtained from commercial sources.

Since libraries of diverse molecules can contain as many as 10¹⁴ to 10¹⁵ different molecules, a screening assay of the invention provides a simple means for identifying those agents in the library that can modulate paraptosis. In particular, a screening assay of the invention can be automated, which allows for high through-put screening of randomly designed libraries of agents to identify those particular agents that can modulate paraptosis.

As described herein, modulation of paraptosis can be a therapeutic strategy for treatment of a variety of neural cell death disorders as well as neoplastic pathologies. As such, administration of a compound that inhibits paraptosis can lead to a reduction in the severity of a neural cell death disorder. As used herein, the term "neural cell death disease" or "neural cell death disorder" refers to any pathological condition that is associated with neural cell death. Neural cell death diseases include neurodegenerative diseases such as retinal degeneration, Huntingtons Disease, Parkinson's Disease and Alzheimer's Disease as well as other diseases associated with the loss of neural cells including, for example, stroke, trauma, global ischemia, hypoxis, seizure-induced excitotoxicity. Exemplary compounds contemplated for inhibiting paraptosis include paraptosis-modulating compounds identified by the invention methods and, for example, acetyl-leucyl-leucyl norleucinal (ALLN), antimycin A and oligomycin.

Administration of a paraptosis-modulating compound that induces paraptosis can lead to a reduction in the severity of a neoplastic disorder. A "neoplastic disorder," as used herein, refers to a condition associated with hyperproliferation of cells and includes benign and malignant expanding lesions of proliferating cells. A benign neoplasm grows in an expansile manner, displacing or compressing surrounding tissues rather than invading them. A malignant neoplasm or cancer, refers to a large group of diseases characterized by uncontrolled growth and spread of abnormal cells and includes any condition of tumors having the properties of anaplasia, invasion, and metastasis.

A paraptosis-modulating compound can be a compound or molecule that binds IGFIR or another paraptosis-mediating molecule with sufficient affinity to modulate paraptosis. A paraptosis-modulating compound can be a macromolecule, such as polypeptide, nucleic acid, carbohydrate or lipid. Thus, a paraptosis-modulating compound can be an antibody, antisense nucleic acid and any compound identified by the methods described below. A paraptosis-modulating compound can also be a derivative, analogue or mimetic compound as well as a small organic compound as long as paraptosis is modulated in the presence of the compound. The size of a paraptosis-modulating compound is not important so long as the molecule exhibits or can be made to exhibit paraptosis-modulating activity. For example, a paraptosis-modulating compound can be as little as between about one and six, and as large as tens or hundreds of monomer building blocks which constitute a macromolecule or chemical binding molecule. Similarly, an organic compound can be a simple or complex structure so long as it has sufficient paraptosis-modulating activity.

Paraptosis-modulating compounds can include, for example, antibodies and other receptor or ligand binding polypeptides of the immune system. Such other molecules of the immune system include for example, T cell receptors (TCR) including CD4 cell receptors.

Additionally, cell surface receptors such as integrins, growth factor receptors and chemokine receptors, as well as any other receptors or fragments thereof that bind to an endogenous paraptosis-mediating molecule such as, for example, IGFIR or caspase-9, or can be made to bind to an endogenous paraptosis-mediating molecule, with sufficient affinity to modulate activity are also paraptosis-modulating compounds useful for practicing the methods of the invention. Examples of selective inhibitors of paraptosis include, for example, IGFIR antisense nucleic acid and transcriptional inhibitors that bind to the IGFIR promoter/regulatory region. Additionally, receptors, ligands, growth factors, cytokines or chemokines, for example, which inhibit the expression of an endogenous paraptosis-mediating molecule are also paraptosis-modulating compounds useful for practicing the methods of the invention. Furthermore, DNA binding polypeptides such as transcription factors and DNA replication factors are likewise included within the definition of the term binding molecule so long as they have selective paraptosis-modulating activity. Finally, polypeptides, nucleic acids and chemical compounds such as those selected from random and combinational libraries can also be paraptosis-modulating compounds.

Various approaches can be used for identifying a paraptosis-modulating compound. For example, one approach is to use the information available regarding the structure and function of caspase-9 and IGFIR to generate binding molecule populations from molecules known to function as protease binding molecules or known to exhibit or be capable of exhibiting binding affinity specific for caspase-9. A paraptosis-modulating compound can be an antibody and other receptor of the immune repertoire. An antibody that recognizes the catalytic site associated with paraptosis is especially useful for practicing the invention method of inhibiting paraptosis by preventing association of caspase-9 and an endogenous paraptosis-mediating molecule. The normal function of such immune receptors is to bind essentially an infinite number of different antigens and ligands. Therefore, generating a diverse population of binding molecules from an immune repertoire, for example, can be useful for identifying a paraptosis-modulating compound.

A paraptosis-modulating compound can further be identified from a large population of unknown molecules by methods well known in the art. Such a population can be a random library of peptides or small molecule compounds. The population can be generated to contain a sufficient diversity of sequence or structure so as to contain a molecule which will bind to an endogenous paraptosis-mediating molecule such as, for example, IGFIR or caspase-9, or their respective nucleic acids. Those skilled in the art will know what size and diversity is necessary or sufficient for the intended purpose. A population of sufficient size and complexity can be generated so as to have a high probability of containing a paraptosis-modulating compound that binds an endogenous paraptosis-mediating molecule such as, for example, IGFIR or caspase-9, with sufficient affinity to modulate activity. Numerous other types of library molecule populations exist and are described further below.

Any molecule that binds to an endogenous paraptosis-mediating molecule, to a gene region that controls expression of such a molecule, or to a regulatory molecule that modulates activity or expression of an endogenous paraptosis-mediating molecule, as well as to any regulatory molecule that modulates IGFIR expression is a paraptosis-modulating compound useful for practicing the invention. For example, a paraptosis-modulating compound can be a regulatory molecule affects an expression of an endogenous paraptosis-mediating molecule such as IGFIR or caspase-9 by modulating the action of a transcription factor that controls or upregulates transcription of the endogenous paraptosis-mediating molecule. In addition, a regulatory molecule that binds with sufficient affinity to a molecule involved in the activation of an endogenous paraptosis-mediating molecule to reduce paraptosis is a paraptosis-modulating compound useful for practicing the methods of the invention.

A paraptosis-modulating compound that can decrease paraptotic activity is useful for reducing the severity of a neural cell death disorder. Conversely, a paraptosis-modulating compound that can induce paraptotic activity is useful for reducing the severity of a neoplastic disorder. A paraptosis-modulating compound can modulate paraptotic activity by binding to an endogenous paraptosis-mediating molecule, to a regulatory molecule that modulates the activity or expression of an endogenous paraptosis-mediating molecule, or to a gene region that controls expression of an endogenous paraptosis-mediating molecule. For example, a paraptosis-modulating compound can be an antibody against a regulator molecule that modulates expression or activity of an endogenous paraptosis-mediating molecule. Alternatively, it may be desired to use populations of random peptide populations to identify further paraptosis-modulating compounds. Those skilled in the art will know or can determine what type of approach and what type of paraptosis-modulating compounds is appropriate.

A moderate sized population for identification of a paraptosis-modulating compound can consist of hundreds and thousands of different binding molecules within the population, whereas a large sized binding molecule population will consist of tens of thousands and millions of different binding molecule species. More specifically, large and diverse populations of binding molecules for the identification of a paraptosis-modulating compound will contain any of about 10⁴, 10⁵, 10⁶, 10⁷, 10⁸, 10⁹, 10¹⁰, or more, different molecule species. One skilled in the art will know the approximate diversity of the population of test-compounds sufficient to identify a paraptosis-modulating compound.

Recombinant libraries of test compounds can be used to identify a paraptosis-modulating compound since large and diverse populations can be rapidly generated and screened via the invention methods. Recombinant libraries of expressed polypeptides useful for identifying a paraptosis-modulating compound can be engineered in a large number of different ways known in the art. Recombinant library methods similarly allow for the production of a large number of test compound populations from naturally occurring repertoires. Whether recombinant or otherwise, essentially any source of test compound population can be used so long as the source provides a sufficient size and diversity of different compounds to identify a paraptosis-modulating compound. If desired, a population of test compounds useful for identifying a paraptosis-modulating compound can be a selectively immobilized to a solid support as described by Watkins et al., Anal. Biochem. 256: 169-177 (1998).

A phage expression library in which lysogenic phage cause the release of bacterially expressed binding molecule polypeptides is a specific example of a recombinant library that can be used to identify a paraptosis-modulating compound. In another type of phage expression library, large numbers of potential paraptosis-modulating compounds can be expressed as fusion polypeptides on the periplasmic surface of bacterial cells. Libraries in yeast and higher eukaryotic cells exist as well and are similarly applicable in the methods of the invention. Those skilled in the art will know or can determine what type of library is useful for identifying a paraptosis-modulating compound.

In addition to the methods described above, which utilize purified polypeptide to screen libraries of compounds for those that modulate paraptosis, a paraptosis-modulating compound can be identified by using purified polypeptide to produce antibodies. For example, antibodies which are specific for IGFIR or another endogenous paraptosis-mediating compound can be used as a paraptosis-modulating compound of the invention and can be generated using methods that are well known in the art. Such paraptosis-modulating compounds can include both polyclonal and monoclonal antibodies against IGFIR, caspase-9, or any endogenous paraptosis-mediating molecule, as well as antigen binding fragments of such antibodies including Fab, F(ab')2, Fd and Fv fragments and the like. In addition, paraptosis-modulating compounds useful for practicing the methods of the invention encompass non-naturally occurring antibodies, including, for example, single chain antibodies, chimeric antibodies, bifunctional antibodies, complementarity determining region-grafted (CDR-grafted) antibodies and humanized antibodies, as well as antigen-binding fragments thereof.

Methods of preparing and isolating antibodies, including polyclonal and monoclonal antibodies, using peptide immunogens, are well known to those skilled in the art and are described, for example, in Harlow and Lane, Antibodies: A Laboratory Manual, Cold Spring Harbor Laboratory Press (1988). Non-naturally occurring antibodies can be constructed using solid phase peptide synthesis, can be produced recombinantly or can be obtained, for example, by screening combinatorial libraries consisting of variable heavy chains and variable light chains as described by Huse et al., Science 246:1275-1281 (1989). These and other methods of making, for example, chimeric, humanized, CDR-grafted, single chain, and bifunctional antibodies are well known to those skilled in the art (Hoogenboom et al., U.S. Patent No. 5,564,332, issued October 15, 1996; Winter and Harris, Immunol. Today 14:243-246 (1993); Ward et al., Nature 341:544-546 (1989) ; Harlow and Lane, Antibodies: A laboratory manual (Cold Spring Harbor Laboratory Press, 1988); Hilyard et al., Protein Engineering: A practical approach (IRL Press 1992); Borrabeck, Antibody Engineering, 2d ed. (Oxford University Press 1995).

A paraptosis-modulating compound can be raised using as an immunogen a substantially purified protein, which can be prepared from natural sources or produced recombinantly, or a peptide portion of a paraptosis-mediating molecule including synthetic peptides. A non-immunogenic peptide portion of a paraptosis-mediating molecule can be made immunogenic by coupling the hapten to a carrier molecule such bovine serum albumin (BSA) or keyhole limpet hemocyanin (KLH), or by expressing the peptide portion as a fusion protein. Various other carrier molecules and methods for coupling a hapten to a carrier molecule are well known in the art (see Harlow and Lane, *supra*, 1988; see, also, Hermanson, Bioconjugate Techniques, Academic Press, 1996). As described above, an antibody paraptosis-modulating compound can also be raised against a regulatory molecule that mediates paraptotic activity.

A paraptosis-modulating compound can be labeled so as to be detectable using methods well known in the art (Hermanson, supra, 1996; Harlow and Lane, supra, 1988; chap. 9). For example, a paraptosis-modulating compound can be linked to a radioisotope or therapeutic agent by methods well known in the art. A paraptosis-modulating compound that directly binds to an endogenous paraptosis-mediating molecule linked to a radioisotope or other moiety capable of visualization can be useful to diagnose or stage the progression of a clinical stage of a neural cell death disorder characterized by the organ or tissue-specific presence or absence of a endogenous paraptosis-mediating molecule.

The invention also provides an in vitro method of inhibiting paraptosis by preventing association of caspase-9 and an endogenous paraptosis-mediating molecule. In a specific embodiment, the invention is directed to a method of identifying a compound that inhibits paraptosis by preventing association between caspase-9 and IGFIR. A method for identifying an agent that alters the association of caspase-9 and an endogenous paraptosis-mediating molecule such as, for example, IGFIR can encompass allowing the caspase-9 protein, or fusion protein to bind to a solid support, then adding IGFIR or IGFIR-IC and an agent to be tested, under conditions suitable for the association of caspase-9 and IGFIR. If desired, caspase-9 or IGFIR can be detectably labeled so as to facilitate identification of the association. Control reactions, which contain or lack either, IGFIR, caspase-9, or the agent, or which substitute the caspase-9 with a dominant negative mutant of caspase-9, which lacks the ability to mediate paraptosis, also can be performed. Following incubation of the reaction mixture, the amount of the IGFIR or IGRIR-IC fragment that is specifically bound to caspase-9 in the presence of an agent can be determined and compared to the amount of binding in the absence of the agent so that agents that modulate the association can be identified. While described with reference to IGFIR, the above methods are applicable for preventing association of caspase-9 and any endogenous paraptosis-mediating molecule.

A transcription activation assay such as the two hybrid assay, which can be performed in yeast cells or mammalian cells, allows the identification of protein-protein interactions and, further, can be particularly useful as the basis for a drug screening assay (Fields and Song, Nature 340:245-246 (1989); Fearon et al., Proc. Natl. Acad: Sci., USA 89:7958-7962 (1992). Such an assay provides the advantage of being performed in cells in culture and, therefore, as compared to an in vitro screening assay, identifies paraptosis-modulating compounds that alter the association of caspase-9 and IGFIR under more physiological conditions provided by a living cell. Such a paraptosis-modulating compound can be identified by detecting an altered level of transcription of a reporter gene, the expression of which is dependent on the association of caspase-9 and IGFIR, as compared to the level of transcription in the absence of the paraptosis-modulating compound.

Where the identification assay using the two hybrid system is performed in yeast, a potential paraptosis-modulating compound may not be able to cross the yeast cell wall and, therefore, cannot enter the yeast cell to alter a protein-protein interaction. The use of yeast spheroplasts, which are yeast cells that lack a cell wall, can circumvent this problem (Smith and Corcoran, Current Protocols in Molecular Biology (ed. Ausubel et al.; Green Publ., NY 1989)). In addition, a paraptosis-modulating compound, upon entering a cell, may require "activation" by a cellular mechanism that may not be present in yeast. Activation of a paraptosis-modulating compound can include, for example, metabolic processing of the agent or a modification such as phosphorylation of the agent, which can be necessary to confer activity upon the paraptosis-modulating compound. In this case, a mammalian cell line can be used to screen a panel of agents (Fearon et al., supra, 1992).

An paraptosis-modulating compound that alters the association of caspase-9 and IGFIR can be useful as a drug to reduce the severity of a pathology characterized by paraptotic activity. Various pathologies are characterized by an increased or decreased level of paraptosis as compared to the normal level of paraptosis for a particular population of cells. For example, decreased levels of paraptosis occur in cancer cells, resulting in the ability of a tumor to form amidst otherwise normal cells in a tissue or organ. In addition, increased levels of paraptosis are associated with a number of neural cell death disorders including stroke, trauma, global ischemia, hypoxis, seizure-induced excitotoxicity, and certain neurodegenerative diseases. Thus, by modulating the association of caspase-9 and IGFIR in cells involved in such pathologies or by modulating paraptosis by an alternative mechanism, improvements can be effected to treatment of the disease.

As disclosed herein, paraptosis can be modulated by contacting the appropriate cell population with a paraptosis-modulating compound, for example, that modulates the action of caspase-9 in mediating paraptosis. Such paraptosis-modulating compounds, therefore, are useful as medicaments for treating a pathology characterized, in part, by aberrant paraptosis. The skilled artisan will recognize the broader usefulness of paraptosis-modulating compounds identified by the methods of the invention for therapeutic treatment of pathologic conditions characterized by aberrant levels of paraptosis.

The invention also is directed to a method of identifying an endogenous paraptosis-mediating molecule, by inducing paraptosis in a cellular system and identifying a molecule that associates with caspase-9, wherein said paraptosis is induced by upregulating caspase-9 in the presence of an apoptotic inhibitors. Subsequent to the induction of paraptosis, a co-immunoprecipitation assay or similar immunoassay can be performed to identify compounds that are associated with caspase-9 in paraptotic cells, as such a compound is a candidate paraptosis-modulating compound. The paraptosis-modulating activity of the caspase-9-co-immunoprecipitating compound can be verified using the methods described herein.

As shown herein, caspase-9 associates with IGFIR in paraptotic cells (Figure 3C). Based on this observation, the invention provides an isolated protein complex encompassing caspase-9 and IGFIR.

In an additional embodiment, the invention provides a compound identified by the invention methods. Such a paraptosis modulating compound can be utilized in treating or reducing the severity of a neoplastic disorder or a neural cell death disorder.

Further disclosed is treating or reducing the severity of a neoplastic disorder by administering a paraptosis inducing compound, as well as treating a neural cell death disorder by administering a paraptosis inhibiting compound.

The paraptosis-modulating compounds can be formulated and administered by those skilled in the art in a manner and in an amount appropriate for the neoplastic disorder or neural cell death disorder to be treated; the rate of disease progression; severity of symptoms, the weight, gender, age and health of the subject; the biochemical nature, bioactivity, bioavailability and side effects of the particular compound; and in a manner compatible with concurrent treatment regimens. An appropriate amount and formulation for decreasing the severity of a neoplastic disorder or neural cell death disorder in humans can be extrapolated from credible animal models known in the art of the particular disorder. It is understood, that the dosage of a paraptosis-modulating compound may have to be adjusted based on the binding affinity of the paraptosis-modulating compound for a second compound, such that a lower dose of a paraptosis-modulating compound exhibiting significantly higher binding affinity can be administered compared to the dosage necessary for a paraptosis-modulating compound with lower binding affinity.

The total amount of a paraptosis-modulating compound can be administered as a single dose or by infusion over a relatively short period of time, or can be administered in multiple doses administered over a more prolonged period of time. Such considerations will depend on a variety of case-specific factors such as, for example, in case of a neurodegenerative disease it will depend on whether the disease category is characterized by acute episodes or gradual neurologic deterioration. For example, for a subject affected with chronic neurologic deterioration the paraptosis-modulating compound can be administered in a slow-release matrice, which can be implanted for systemic delivery or at the site of the target tissue. Contemplated matrices useful for controlled release of therapeutic compounds are well known in the art, and include materials such as DepoFoamTM, biopolymers, micropumps, and the like.

The paraptosis-modulating compounds of the invention can be administered to the subject by any number of routes known in the art including, for example, systemically, such as intravenously or intraarterially. An paraptosis-modulating compound can be provided, for example, in the form of isolated and substantially purified polypetides and polypeptide fragments in pharmaceutically acceptable formulations using formulation methods known to those of ordinary skill in the art. These formulations can be administered by standard routes, including, for example, topical, transdermal, intraperitoneal, intracranial, intracerebroventricular, intracerebral, intravaginal, intrauterine, oral, rectal or parenteral (e.g., intravenous, intraspinal, subcutaneous or intramuscular) routes. In addition, a paraptosis-modulating compound can be incorporated into biodegradable polymers allowing for sustained release of the compound useful for reducing the severity of a neoplastic disorder or neural cell death disorder. Biodegradable polymers and their use are described, for example, in Brem et al., J. Neurosurg. 74:441-446 (1991).

A paraptosis-modulating compound can be administered as a solution or suspension together with a pharmaceutically acceptable medium. Such a pharmaceutically acceptable medium can be, for example, sterile aqueous solvents such as sodium phosphate buffer, phosphate buffered saline, normal saline or Ringer's solution or other physiologically buffered saline, or other solvent or vehicle such as a glycol, glycerol, an oil such as olive oil or an injectable organic ester. A pharmaceutically acceptable medium can additionally contain physiologically acceptable compounds that act, for example, stabilize the paraptosis-modulating compound, increase its solubility, or increase its absorption. Such physiologically acceptable compounds include, for example, carbohydrates such as glucose, sucrose or dextrans; antioxidants such as ascorbic acid or glutathione; receptor mediated permeabilizers, which can be used to increase permeability of the blood-brain barrier; chelating agents such as EDTA, which disrupts microbial membranes; divalent metal ions such as calcium or magnesium; low molecular weight proteins; lipids or liposomes; or other stabilizers or excipients. Those skilled in the art understand that the choice of a pharmaceutically acceptable carrier depends on the route of administration of the compound containing the paraptosis-modulating compound and on its particular physical and chemical characteristics.

Formulations suitable for parenteral administration include aqueous and non-aqueous sterile injection solutions such as the pharmaceutically acceptable mediums described above. The solutions can additionally contain, for example, buffers, bacteriostats and solutes which render the formulation isotonic with the blood of the intended recipient. Other formulations include, for example, aqueous and non-aqueous sterile suspensions which can include suspending agents and thickening agents. The formulations can be presented in unit-dose or multi-dose containers, for example, sealed ampules and vials, and can be stored in a lyophilized condition requiring, for example, the addition of the sterile liquid carrier, immediately prior to use. Extemporaneous injection solutions and suspensions can be prepared from sterile powders, granules and tablets of the kind previously described.

For applications that require the compounds and compositions to cross the blood-brain barrier, formulations that increase the lipophilicity of the compound are particularly desirable. For example, the paraptosis-modulating compound can be incorporated into liposomes (Gregoriadis, Liposome Technology, Vols. I to III, 2nd ed. (CRC Press, Boca Raton FL (1993)). Liposomes, which consist of phospholipids or other lipids, are nontoxic, physiologically acceptable and metabolizable carriers that are relatively simple to make and administer.

A paraptosis-modulating compound can also be prepared as nanoparticles. Adsorbing peptide compounds onto the surface of nanoparticles has proven effective in delivering peptide drugs to the brain (see Kreuter et al., Brain Res. 674:171-174 (1995)). Exemplary nanoparticles are colloidal polymer particles of poly-butylcyanoacrylate with paraptosis-modulating compound adsorbed onto the surface and then coated with polysorbate 80.

Image-guided ultrasound delivery of a paraptosis-modulating compound through the blood-brain barrier to selected locations in the brain can be utilized as described in U.S. Patent No. 5,752,515. Briefly, to deliver a paraptosis-modulating compound past the blood-brain barrier a selected location in the brain is targeted and ultrasound used to induce a change detectable by imaging in the central nervous system (CNS) tissues and/or fluids at that location. At least a portion of the brain in the vicinity of the selected location is imaged, for example, via magnetic resonance imaging (MRI), to confirm the location of the change. An paraptosis-modulating compound in the patient's bloodstream can delivered to the confirmed location by applying ultrasound to effect opening of the blood-brain barrier at that location and, thereby, to induce uptake of the paraptosis-modulating compound.

In addition, polypeptides called receptor mediated permeabilizers (RMP) can be used to increase the permeability of the blood-brain barrier to molecules such as therapeutic agents or diagnostic agents as described in U.S. Patent Nos. 5,268,164; 5,506,206; and 5,686,416. These receptor mediated permeabilizers can be intravenously co-administered to a host with molecules whose desired destination is the cerebrospinal fluid compartment of the brain. The permeabilizer polypeptides or conformational analogues thereof allow therapeutic agents to penetrate the blood-brain barrier and arrive at their target destination.

In current treatment regimes for neoplastic disorders as well as those for neural cell death disorders, more than one compound is often administered to an individual for management of the same or different aspects of the disease. Similarly, a paraptosis-modulating compound can advantageously be formulated with a second therapeutic compound such as an anti-inflammatory compound, immunosuppressive compound or any other compound that manages the same or different aspects of the disease. Such compounds include, for example, methylprednisolone acetate, dexamethasone and betamethasone. Methods of reducing the severity of a neoplastic disorder or neural' cell death disorder include administering a paraptosis-modulating compound alone, in combination with, or in sequence with, such other compounds. Alternatively, combination therapies can consist of fusion proteins, where the paraptosis-modulating compound is linked to a heterologous protein, such as a therapeutic protein.

The following example is intended to illustrate but not limit the present invention,

### EXAMPLE I

### Insulin-Like Growth Factor Receptor (IGFIR) Induces Nonapoptotic Cell Death

This example demonstrates that expression of the insulin-like growth factor I receptor (IGFIR) induces nonapoptotic programmed cell death.

The intracellular domain of IGFIR, designated IGFIR-IC was expressed in 293T cells, 293, MCF-7, Cos-7, and primary cultures of mouse embryonic fibroblasts. Cells were cultured in DMEM containing 10% FBS and 1% penicillin/streptomycin.

To obtain the wild-type IGFIR-IC construct, the human IGFIR β subunit intracellular domain was subcloned from a NT2 (human teratocarcinoma) cDNA library by PCR amplification and inserted into pcDNA3 (Invitrogen, Carlsbad, CA). Fifteen amino acids containing the Src myristylation signal sequence were incorporated into the construct.The sequences of all constructs were confirmed and Western blot analyses were performed to verify protein expression.

Transient transfections used Lipofect-Amine (GIBCO, BRL, Rockville, MD) according to the manufacturer's instructions. Briefly, 1X10⁶ 293T cells were seeded in 6 cm dishes, and transfected the next day using a ratio of DNA:Lipofect-Amine of 1µg:5µl. Transfection efficiency was 60-80% for 293T, as determined by X-gal staining after transfection of a b-galactosidase construct.

Following expression of the IGIFR-IC constructs, the cell cultures were assayed for cell death. Cell death was assessed by trypan blue exclusion. Floating cells were collected at the indicated times after transfection, by centrifugation for 10 minutes at 1500 rpm. The cell pellet was subsequently resuspended in 50 µl of PBS, to which 50 µl of trypan blue was added. Dead cells were counted by 4 independent hemocytometer counts.

Expression of IGFIR-IC in 293T cells, 293 Cells, MCF-7 cells, Cos-7 cells and primary cultures of mouse embryonic fibroblasts induced cell death that did not fit the criteria for apoptosis. This demonstrates that the ability of IGFIR to induce this alternate type of cell death is not restricted to a specific cell type. Overexpression of full length IGFIR in 293 cells also induced cell death reproducing the same morphology observed with the IC domain although with a much weaker effect than that observed with the IC domain.

Omission of the myristylation site in the IGFIR-IC construct destroyed its ability to induce cell death (Figure 1A). Mutagenic analysis was performed to exclude the possibility that the cell death induction was a nonspecific effect of the overexpression of a membrane-targeted protein. Mutants of IGFIR-IC were obtained by QuikChange mutagenesis (Stratagene, La Jolla, CA) from the wild type construct. The sequences of all constructs were confirmed and Western blot analyses were performed to verify protein expression. The presence of both the kinase domain and non-catalytic carboxy-terminus proved to be necessary to induce cell death (Figure 1A). Thus, cell death is a specific effect of membrane-targeted IGFIR.

### IGFIR-Induced Cell Death is Nonapototic

Dead 293T cells were further studied to ascertain the nature of the IGFIR-IC induced cell death. Samples for electron microscopy were prepared as described in Bozzola and Russell, Electron Microscopy (Jones and Barlett, 1992), and examined on a Hitachi 600 electron microscope. Ultrastructurally, cell death induced by IGFIR-IC in 293T cells lacked the features of apoptosis that were present in the caspase-9 transfected cells, including nuclear fragmentation, apoptotic body formation, and chromatin condensation (Figure 1C). In lieu of these characteristics, the IGFIR-IC transfected cells showed cytoplasmic vacuolation (Figures 1B and 1C). Vacuoles were derived predominantly from the endoplasmic reticulum, but mitochondrial swelling was also observed. Autophagic vacuoles were not observed.

Biochemically, caspase enzymatic assays showed that, while the pro-apoptotic protein Bax induced cell death with associated caspase activation, IGFIR-IC induced cell death without associated caspase activation (Figure 5). Briefly, caspase-3 activity was measured 48 hours following transfection of 293T cells with the indicated expression constructs. CPP32 (caspase-3)-like activity was determined by using the ApoAlert Caspase-3 Assay Kit (Clontech, Palo Alto, CA) according to manufacturers instructions. Expression of Bax, but not IGFIR-IC, resulted in the induction of caspase-3 activity. Similar results were obtained when the caspase assays were performed 24 hours after transfection. Thus, the pro-apoptotic protein Bax induced cell death with associated caspase activation, IGFIR-IC induced cell death without associated caspase activation.

Since one of the defining features of apoptotic cell death is the fragmentation of chromatin, genome integrity was assayed by agarose gel electrophoresis. Oligonucleosomal DNA fragmentation was assessed by extraction of soluble DNA from 3X10⁶ transfected cells as by Frisch and Francis, Journal of Cell Biology 124:619-626 (1994). As shown in Figure 2A, fragments suggestive of internucleosomal DNA cleavage were observed in the control cells transfected with a Bax expression construct, but were absent in the IGFIR-IC transfected cells.

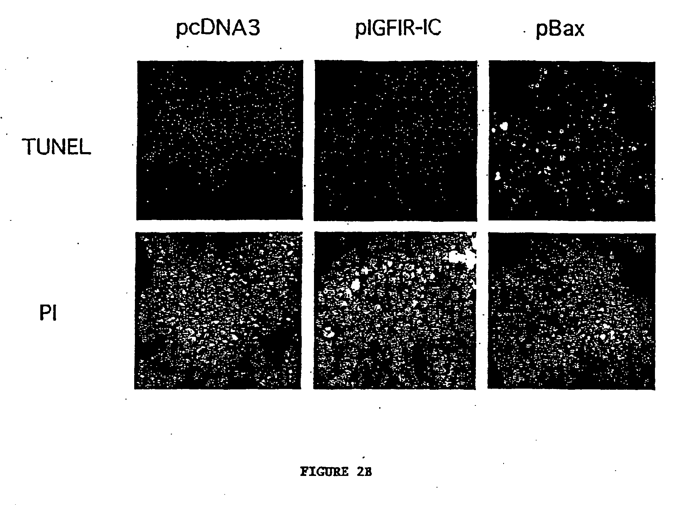

Terminal deoxynucleotidyl transferase-mediated dUTP nick-end labeling (TUNEL) staining of cells transfected with either IGFIR-IC or Bax was performed by using the Apoptosis Detection System (Fluorescein) from Promega (Madison, WI) according to manufacturer's instructions. As shown in Figure 2B, the Bax-transfected cells were labeled in the TUNEL assay, while those cells transfected with IGFIR-IC were not labeled.

The results obtained with apoptosis inhibitors supported the ultrastructural and biochemical results described above. Briefly, caspase inhibitor BAF was added to pIGFIR-IC transfected 293T cells at a concentration of 50 mM. In addition, 293T cells were cotransfected with 0.5 mg of pIGFIR-IC and 1.5 mg of either pcDNA3 or pxiap or pBcl-xL. As shown in Table 1 and Figure 6, neither the caspase inhibitors Benzyloxycarbonyl-valyl-alanyl-aspartyl fluoromethyl ketone (zVAD.fmk), t-butyloxycarbonyl-Asp(O-methyl)-fluoromethyl ketone (BAF), p35, and X-chromosome-linked inhibitor of apaoptosis (xiap), nor the Hcl-2 family member Bcl-x_{L} inhibited 293T cell death induced by IGFIR-IC. BAF and zVAD.fmk were obtained from Enzyme System Products (Livermore, CA).

| **Table 1.** Comparison of Apoptosis, Necrosis and Paraptosis | | | |
|---|---|---|---|
| | **Apoptosis** | **Necrosis** | **Paraptosis** |
| **Morphology** | | | |
| Nuclear fragmentation | + | - | - |
| Chromatin **condensation** | + | - | ± |
| Apoptotic bodies | + | - | - |
| Cytoplasmic vacuolation | - | + | + |
| Mitochondrial swelling | Sometimes | + | Late |

| **Genomic effect** | | | |
|---|---|---|---|
| TUNEL | + | Usually - | - |
| Internucleosomal DNA fragmentation | + | - | - |

| **Caspase activity** | | | |
|---|---|---|---|
| DEVD-cleaving activity | + | - | - |
| Casp-3 processing | + | - | - |
| **PARP cleavage** | + 85kd fragment | + 50-62 kd fragments | - |

| **Inhibition by:** | | | |
|---|---|---|---|
| zVAD.fmk | + | - | - |
| Boc-Asp.fmk (BAF) | + | - | - |
| p35 | + | - | - |
| xiap | + | - | - |
| Bcl-x_{L} | + | Usually - | - |
| Actinomycin D | Sometimes | - | + |
| Cycloheximide | Sometimes | - | 0 |

### Cell Death Induced by IGPIR Requires Protein Synthesis

To assess IGFIR-induced cell death following treatment with Actinomycin D and cycloheximide, both drugs were obtained form Sigma and added to IGFIR-IC transfected 293T cells. Briefly, 293T cells were transfected as described above and 8.5 hours after the beginning of transfection the drugs were added at the indicated concentrations. The drugs were subsequently removed after 12 hours and the cells were fed with fresh medium. Floating cells were collected 24 hrs after the removal of the drugs and dead cells were counted in the presence of trypan blue. These results demonstrate that IGFIR-driven cell death requires transcription and translation. As shown in Figure 2C, Actinomycin D restored cell viability to control levels after transfection with IGFIR-IC in 293T cells. Furthermore, treatment with cycloheximide obtained form Sigma induced a dose-dependent block of IGFIR-induced cell death.

Further support IGFIR-induced cell death is distinct from necrosis was provided by a difference in cleavage of poly-(ADP-ribose) polymerase (PARP), which results in 50-62 kd fragments in necrosis, but not in paraptosis; and by the finding that oligomycin (100ng/ml), which blocks Bax-induced apoptosis, blocked the phenotype.

### Caspase-9 Mediates IGFIR-induced Cell Death

To assess whether caspase-9 has a functional role in IGFIR-induced cell death 293T cells were transfected with 2µg of DNA consisting of either pcDNA3, 0.5 µg pIGFIR-IC + 1.5 µg pcDNA3 (Invitrogen, Carlsbad, CA), or 0.5 µg pIGFIR-IC + 1.5 µg dominant negative caspase-9 (pC9DN) or dominant negative caspase-7 (pC7DN) expression constructs. 48 hours after transfection, dead cells were counted. As shown in Figure 3A, the catalytic mutant of caspase-9 zymogen (pC9DN), which acts as a dominant negative form of pro-caspase-9 described by Yuan et al., Cell 75, 641-52 (1993); Rehemtulla et al., J Biol Chem 272:25783-6 (1997); Friedlander et al., J Exp Med 185:933-40 (1997); Li et al., Cell 91: 479-89 (1997), blocked IGFIR-IC-induced cell death. This effect was specific for caspase-9 as similar forms of caspases-2, 3, 6, 7, and 8 had no effect (see Figures 3A and 3B).

To further characterize the interaction between caspase-9 and IGFIR, cell lysis and immunoprecipitation were performed as described in Ye et al., Journal of Biological Chemistry 274:30202-30208 (1999). Briefly, protein extracts from cells co-transfected with pIGFIR-IC wild-type or mutants, as indicated in Figure 3C, and FLAG-tagged C9DN expression constructs were subjected to immunoprecipitation using an anti-IGFIR polyclonal antibody. Immunoprecipitates were subsequently resolved by 15% SDS-PAGE and Western blotting was performed using a monoclonal antibody against the FLAG epitope to detect caspase-9. As shown in Figure 3C, caspase-9 coimmunoprecipitated with IGFIR-IC, suggesting that the recruitment of caspase-9 by IGFIR may trigger the cell death stimulus.

Furthermore, consistent with the possibility for a functional role of caspase-9 in this alternative form of cell death, neither the IGFIR-IC construct missing the membrane recruitment signal, nor the truncated forms previously described that failed to induce cell death, coimmunoprecipitated with caspase-9 (Figure 3C), whereas the deletion mutant in the C-terminus (D1264-1276), which retained cell death-promoting activity, also coimmunoprecipitated with caspase-9.

### Caspase-9 Induces Both Apoptosis and Nonapoptotic Cell Death

To further investigate the role of caspase-9 or caspase-9 zymogen as a mediator of both apoptotic and non-apoptotic programmed cell death, caspase-9 was expressed in the absence of IGFIR-IC. 293T cells were transfected with the indicated pcDNA3-based caspase zymogen expression constructs. As shown in Figure 4, both apoptosis and non-apoptotic programmed cell death were induced, with apoptosis representing approximately 80-90% of the cell death.

In a parallel assay, the general caspase inhibitor BAF was added at 50 µM final concentration. Cell death was assayed 48 hours after transfection. While BAF was able to inhibit caspase-7 induced cell death completely, it only inhibited caspase-9 induced cell death partially (30%). However, when apoptosis was inhibited by BAF, virtually all of the apoptosis was eliminated, revealing the non-apoptotic cell death more clearly (Figures 4A and 4B). Furthermore, all of the features of cell death induced by IGFIR-IC were reproduced by the expression of caspase-9 in the presence of BAF (see Figure 4 and Table 1). In contrast, cell death induced by the expression of caspase-7 was completely eliminated by BAF (Figure 4A).

In addition to separating the pro-apoptotic and nonapoptotic effects of caspase-9 by adding BAF, these effects could also be distinguished by expression in Apaf-1 null cells, which have been shown previously to be resistant to a variety of apoptotic stimuli by Yoshida et al., Cell 94:739-50 (1998). Apaf-1 null mouse embryonic fibroblasts were cultured in DMEM containing 10% FBS and 1% penicillin/streptomycin. Transient transfections used Lipofect-Amine (GIBCO, BRL) according to the manufacturers instructions. 2x10⁵ Apaf-1 null cells were seeded in 6 cm dishes, and transfected the next day using a ratio of DNA:Lipofect-Amine of 1µg:5µl. Transfection efficiency was 10-20% for the Apaf-1 KO cells as determined by X-gal staining after transfection of a β-galactosidase construct. Both floating and adherent cells were collected by trypsinization and cell death was determined as the percentage of dead cells over the total number of cells.

As shown in Figure 7, cell death was induced both by IGFIR-IC and caspase-9 expression in Apaf-1 null cells in the presence of BAF. In addition, the morphological features of non-apoptotic cell death described above were also reproduced in Apaf-1 null cells.

To further distinguish the pro-apoptotic and nonapoptotic effects of caspase-9, the effect of two mutations of the zymogen processing sites within caspase-9 zymogen, D315 and D330, were studied. Expression of these mutants, which block processing of caspase-9, reduced the percentage of dead cells due to apoptosis from 85±6% in the wild-type to 36±8% in the double mutant, with a concomitant increase in non-apoptotic cell death.

## Claims

1. An *in vitro* method for identifying a paraptosis-modulating compound, said method comprising:
(a) inducing paraptosis in a cellular system;
(b) contacting said cellular system with a test-compound; and
(c) identifying a compound that modulates paraptosis,
wherein said paraptosis is induced by upregulating caspase-9 in the presence of an apoptosis inhibitor.

2. An *in vitro* method of identifying a paraptosis-modulating compound, said method comprising
(a) inducing paraptosis in a first cellular system and inducing paraptosis in a second cellular system;
(b) contacting said first cellular system with a test-compound and contacting said second cellular system with a control-compound; and
(c) comparing the amount of cell death between said first and said second cellular system, wherein a difference in the amount of cell death between said first and said second cellular system indicates that said test compound modulates paraptosis,
wherein said paraptosis in said first and said second cellular system is induced by upregulating caspase-9 in the presence of an apoptosis inhibitor.

3. The method of claim 1 or 2, wherein said test compound induces paraptosis.

4. The method of claim 1 or 2, wherein said test compound inhibits paraptosis.

5. The method of claim 4, wherein said test compound inhibits paraptosis by preventing association between caspase-9 and an endogenous paraptosis-mediating molecule.

6. The method of claim 5, wherein said endogenous paraptosis-mediating molecule is Insulin-Like Growth Factor I Receptor (IGFIR).

7. A method of identifying an endogenous paraptosis mediating molecule, said method comprising
a) inducing paraptosis in a cellular system; and
b) identifying a molecule that associates with caspase-9,
wherein said paraptosis is induced by upregulating caspase-9 in the presence of an apoptosis inhibitor.

8. An isolated protein complex, comprising caspase-9 and Insulin Like Growth Factor I Receptor (IGFIR).

9. An *in vitro* method of inhibiting paraptosis, said method comprising contacting a cellular system with a compound selected from the group consisting of acetyl-leucyl-leucyl norleucinal (ALLN), antimycin A and oligomycin.

## Patentansprüche

1. Ein *in vitro* Verfahren zur Identifizierung einer Verbindung, welche Paraptose moduliert, wobei das Verfahren Schritte umfasst, bei denen man:
(a) in einem zellulären System Paraptose induziert;
(b) das zelluläre Systeme mit einer Testverbindung in Kontakt bringt; und
(c) eine Verbindung identifiziert, welche Paraptose moduliert,
wobei die Paraptose induziert wird, indem man Caspase-9 in der Anwesenheit eines Apoptose-Inhibitors hoch reguliert.

2. Ein *in vitro* Verfahren zur Identifizierung einer Verbindung, welche Paraptose moduliert, wobei das Verfahren Schritte umfasst, bei denen man:
(a) in einem ersten zellulären System Paraptose induziert und in einem zweiten zellulären System Paraptose induziert;
(b) das erste zelluläre System mit einer Testverbindung in Kontakt bringt und das zweite zelluläre System mit einer Kontrollverbindung in Kontakt bringt; und
(c) die Menge an Zelltod zwischen dem ersten und dem zweiten zellulären System vergleicht, wobei ein Unterschied bei der Menge an Zelltod zwischen dem ersten und dem zweiten zellulären Systems darauf hinweist, dass die Testverbindung Paraptose moduliert,
wobei die Paraptose in dem ersten und dem zweiten zellulären System induziert wird, indem man Caspase-9 in der Anwesenheit eines Apoptose-Inhibitors hoch reguliert.

3. Das Verfahren nach Anspruch 1 oder 2, **dadurch** gekennzeicnet, dass die Testverbindung Paraptose induziert.

4. Das Verfahren nach Anspruch 1 oder 2, **dadurch gekennzeichnet, dass** die Testverbindung Paraptose inhibiert.

5. Das Verfahre nach Anspruch 4, **dadurch gekennzeichnet, dass** die Testverbindung Paraptose inhibiert, indem sie eine Assoziation zwischen Caspase-9 und einem endogenen Paraptose vermittelnden Molekül verhindert.

6. Das Verfahren nach Anspruch 5, **dadurch gekennzeichnet, dass** das endogene Paraptose vermittelnde Molekül Insulin-artiger Wachstumsfaktor I Rezeptor (Insulin-Like Growth Factor I Receptor, IGFIR) ist.

7. Ein Verfahren zur Identifizierung eines endogenen Paraptose vermittelnden Moleküls, wobei das Verfahren Schritte umfasst, bei denen man:
(a) in einen zellulären System Paraptose induziert; und
(b) ein Molekül identifiziert, welches mit Caspase-9 assoziiert,
wobei die Paraptose induziert wird, in dem man Caspase-9 in der Anwesenheit eines Apoptose-Inhibitors hoch reguliert.

8. Ein isolierter Proteinkomplex, umfassend Caspase-9 und Insulin-artigen Wachstumsfaktor I Rezeptor (Insulin-Like Growth Factor I Receptor, IGFIR).

9. Ein *in vitro* Verfahren zur Inhibierung von Paraptose, wobei das Verfahren es umfasst, dass man ein zelluläres System mit einer Verbindung ausgewählt aus der Gruppe bestehend aus Acetyl-Leucyl-Leucyl-Norleucinal (ALLN), Antimycin A und Oligomycin, in Kontakt bringt.

## Revendications

1. Procédé *in vitro* d'identification d'un composé modulateur de paraptose, lequel procédé comprend les étapes suivantes :
a) induire la paraptose au sein d'un système cellulaire ;
b) mettre ce système cellulaire en contact avec un composé d'essai ;
c) et identifier un composé qui module la paraptose,
et dans lequel ladite paraptose est induite par régulation à la hausse de la caspase-9 en présence d'un inhibiteur d'apoptose.

2. Procédé *in vitro* d'identification d'un composé modulateur de paraptose, lequel procédé comprend les étapes suivantes :
a) induire la paraptose au sein d'un premier système cellulaire et induire la paraptose au sein d'un deuxième système cellulaire ;
b) mettre ledit premier système cellulaire en contact avec un composé d'essai et mettre ledit deuxième système cellulaire en contact avec un composé témoin ;
c) et comparer les nombres de cellules mortes dans lesdits premier et deuxième systèmes cellulaires, étant entendu qu'une différence entre les nombres de cellules mortes dans lesdits premier et deuxième systèmes cellulaires indique que le composé d'essai est un modulateur de paraptose,
et dans lequel ladite paraptose est induite dans lesdits premier et deuxième systèmes cellulaires par régulation à la hausse de la caspase-9 en présence d'un inhibiteur d'apoptose.

3. Procédé conforme à la revendication 1 ou 2, dans lequel ledit composé d'essai induit la paraptose.

4. Procédé conforme à la revendication 1 ou 2, dans lequel ledit composé d'essai inhibe la paraptose.

5. Procédé conforme à la revendication 4, dans lequel ledit composé d'essai inhibe la paraptose en empêchant l'association de la caspase-9 et d'une molécule endogène médiant la paraptose.

6. Procédé conforme à la revendication 5, dans lequel ladite molécule endogène médiant la paraptose est le récepteur IGFIR (récepteur du facteur de croissance 1 analogue à l'insuline).

7. Procédé d'identification d'une molécule endogène médiant la paraptose, lequel procédé comprend les étapes suivantes :
a) induire la paraptose au sein d'un système cellulaire ;
b) et identifier une molécule qui s'associe avec la caspase-9 ;
et dans lequel ladite paraptose est induite par régulation à la hausse de la caspase-9 en présence d'un inhibiteur d'apoptose.

8. Complexe protéique isolé, comprenant de la caspase-9 et du récepteur IGFIR (récepteur du facteur de croissance I analogue à l'insuline).

9. Procédé *in vitro* d'inhibition de la paraptose, lequel procédé comprend le fait de mettre un système cellulaire en contact avec un composé choisi dans l'ensemble formé par l'acétyl-leucyl-leucyl-norleucinal (ALLN), l'antimycine A et l'oligomycine.
